# EUROPÄISCHE PATENTSCHRIFT

(11) **EP 2 388 596 B1**
(45) Veröffentlichungstag und Bekanntmachung des Hinweises auf die Patenterteilung: **22.05.2013**
(21) Anmeldenummer: 11002342.1
(22) Anmeldetag: 03.07.2008
(51) Int. Cl.: G01N 33/86, C12Q 1/37

(54) **Verfahren zur Bestimmung der von Willebrand Faktor-spaltenden Aktivität der ADAMTS-13-Protease**
Process for the determination of the von Willebrand factor-cleaving activity of the ADAMTS-13 protease
Procédé pour la détermination de l'activité de clivage du facteur von Willebrand de la protéase ADAMTS-13

(30) Priorität: 06.07.2007 DE 102007031708
(43) Veröffentlichungstag der Anmeldung: 23.11.2011
(62) Teilanmeldung aus: 08773827.4
(73) Patentinhaber: Siemens Healthcare Diagnostics Products GmbH, 35041 Marburg (DE)
(72) Erfinder: Althaus, Harald, 35083 Wetter (DE); Obser, Tobias, 22455 Hamburg (DE); Patzke, Jürgen, Dr., 35043 Marburg (DE); Schneppenheim, Reinhard Prof., 22399 Hamburg (DE)

(56) Entgegenhaltungen:
- WO-A2-01/02853
- US-A1- 2005 186 646
- HASSENPFLUG WOLF ACHIM ET AL: "Impact of mutations in the von Willebrand factor A2 domain on ADAMTS13-dependent proteolysis.", BLOOD 15 MAR 2006, Bd. 107, Nr. 6, 15. März 2006 (2006-03-15) , Seiten 2339-2345, XP002520836, ISSN: 0006-4971
- RAYES J ET AL: "Effect of von Willebrand disease type 2B and type 2M mutations on the susceptibility of von Willebrand factor to ADAMTS-13.", JOURNAL OF THROMBOSIS AND HAEMOSTASIS : JTH FEB 2007, Bd. 5, Nr. 2, Februar 2007 (2007-02), Seiten 321-328, XP002520837, ISSN: 1538-7933
- KOSTOUSOV ET AL: "Novel, semi-automated, 60-min-assay to determine von Willebrand factor cleaving activity of ADAMTS-13", THROMBOSIS RESEARCH, TARRYTOWN, NY, US, Bd. 118, Nr. 6, 30. Oktober 2006 (2006-10-30), Seiten 723-731, XP025103316, ISSN: 0049-3848
- BISWAS TAPAN K ET AL: "Properties of soluble his-tagged rGPIb alpha(1-483) wild-type and increase-of-function mutants.", BLOOD, Bd. 106, Nr. 11, Part 2, November 2005 (2005-11), Seite 67B, XP002512023, & 47TH ANNUAL MEETING OF THE AMERICAN-SOCIETY-OF-HEMATOLOGY; ATLANTA, GA, USA; DECEMBER 10 -13, 2005 ISSN: 0006-4971
- TAIT A SASHA ET AL: "Phenotype changes resulting in high-affinity binding of von Willebrand factor to recombinant glycoprotein Ib-IX: Analysis of the platelet-type von Willebrand disease mutations", BLOOD, Bd. 98, Nr. 6, 15. September 2001 (2001-09-15), Seiten 1812-1818, XP002512024, ISSN: 0006-4971
- FEDERICI A B ET AL: "A sensitive ristocetin co-factor activity assay with recombinant glycoprotein Ib[alpha] for the diagnosis of patients with low von Willebrand factor levels", HAEMATOLOGICA, FONDAZIONE FERRATA STORTI, ROME, IT, Bd. 89, Nr. 1, 1. Januar 2004 (2004-01-01) , Seiten 77-85, XP002390136, ISSN: 0390-6078
- VANHOORELBEKE KAREN ET AL: "A reliable and reproducible ELISA method to measure ristocetin cofactor activity of von Willebrand factor", THROMBOSIS AND HAEMOSTASIS, STUTTGART, DE, Bd. 83, Nr. 1, 1. Januar 2000 (2000-01-01) , Seiten 107-113, XP000974940, ISSN: 0340-6245
- TRIPODI A ET AL: "Measurement of von Willebrand factor cleaving protease (ADAMTS-13): results of an international collaborative study involving 11 methods testing the same set of coded plasmas.", JOURNAL OF THROMBOSIS AND HAEMOSTASIS : JTH SEP 2004, Bd. 2, Nr. 9, September 2004 (2004-09), Seiten 1601-1609, XP002520838, ISSN: 1538-7933
- KITAGUCHI T ET AL: "CHARACTERIZATION OF LIPOSOMES CARRYING VON WILLEBRAND FACTOR-BINDING DOMAIN OF PLATELET GLYCOPROTEIN IBALPHA: A POTENTIALSUBSTITUTE FOR PLATELET TRANSFUSION", BIOCHEMICAL AND BIOPHYSICAL RESEARCH COMMUNICATIONS, ACADEMIC PRESS INC. ORLANDO, FL, US, Bd. 261, Nr. 3, 11. August 1999 (1999-08-11), Seiten 784-789, XP001019106, ISSN: 0006-291X, DOI: 10.1006/BBRC.1999.1088

## Beschreibung

Die Erfindung liegt auf dem Gebiet der Gerinnungsdiagnostik und betrifft ein Verfahren zur Bestimmung von Willebrand Faktor (VWF)-spaltenden Aktivität der ADAMTS-13-Protease.

ADAMTS-13 (a disintegrin and metalloprotease with thrombospondin motifs) ist eine Metalloprotease, die den von Willebrand Faktor (VWF) proteolytisch spaltet und dadurch seine Aktivität verringert. Es sind congenitale sowie erworbene Defizienzen der ADAMTS-13-Enzymaktivität bekannt, die verschiedene Erkrankungen verursachen, wie z. B. die thrombotische, thrombozytopenische Purpura (TTP), eine lebensbedrohliche thromboembolische, die Mikrozirkulation betreffende Erkrankung. Im Plasma von TTP-Patienten werden ungewöhnlich große Multimere des VWF gefunden und als Ursache für die Bildung von VWF- und Thrombozyten-reichen Thromben angesehen. Die Bestimmung der VWFspaltenden Proteaseaktivität der ADAMTS-13-Protease in Patientenproben ist daher von diagnostischer Bedeutung.

Im Stand der Technik sind verschiedene Verfahren zur Bestimmung der VWFspaltenden Aktivität der ADAMTS-13-Protease bzw. für die Diagnose eines ADAMTS-13-Mangels bekannt:

Furlan et al. (1996) beschreiben ein Verfahren, bei dem eine Probe, die die ADAMTS-13-Protease enthält, mit gereinigtem humanen VWF als Substrat inkubiert wird, und wobei der Nachweis der proteolytischen Aktivität der ADAMTS-13-Protease durch eine anschließende VWF-Multimeranalyse mittels SDS-Agarosegelelektrophorese oder durch eine VWF-Bruchstückanalyse mittels SDS-Polyacrylamidgelelektrophorese (SDS-PAGE) und anschließendem Immunoblotting erfolgt [Furlan, M., Robles, R. and Lämmle, B. (1996) Partial purification and characterization of a protease from human plasma cleaving von Willebrand factor to fragments produced by in vivo proteolysis. Blood 87, 10: 4223-4234]. Derartige Multimeranalysen mittels Gelelektrophorese sind extrem arbeits- und zeitaufwändig und daher nicht für den Einsatz im klinischen Labor geeignet.

Andere funktionelle Testverfahren bestimmen die proteolytische Aktivität der ADAMTS-13-Protease, indem die Probe, die die ADAMTS-13-Protease enthält, mit VWF als Substrat inkubiert wird, und anschließend der Aktivitätsverlust des VWF-Substrats bestimmt wird. Je mehr ADAMTS-13-Protease in der Probe enthalten ist, desto mehr VWF-Substrat wird gespalten und desto weniger VWF-Aktivität kann anschließend im Reaktionsgemisch nachgewiesen werden. Der Vorteil dieser funktionellen Verfahren besteht darin, dass durch die Verwendung des VWF als Substrat und der Messung des Funktionsverlustes der großen Multimere die in vivo relevante Funktion der ADAMTS-13-Protease in vitro simuliert wird.

WO 2004/005451 A2 offenbart ein Verfahren, bei dem eine Probe, die die ADAMTS-13-Protease enthält, mit gereinigtem humanem VWF als Substrat inkubiert wird, und wobei der Nachweis der proteolytischen Aktivität der ADAMTS-13-Protease durch eine anschließende Bestimmung der im Reaktionsgemisch verbliebenen VWF-Aktivität mittels eines Ristocetin-Kofaktor-Testes erfolgt. Alternativ kann die im Reaktionsgemisch verbliebene VWF-Aktivität mittels eines Kollagenbindungstestes erfolgen (siehe z. B. WO 00/50904). Nachteilig ist, dass das Reaktionsgemisch sehr lange inkubiert werden muss, zum Teil über Nacht, um einen ausreichenden proteolytischen Abbau des VWF zu bewirken. Ein verbessertes Verfahren, bei dem die Inkubationszeit auf 60 Minuten reduziert ist, ist beschrieben in Kostousov, V., Fehr, J., Bombeli, T. (2006) Novel, semiautomated, 60-min-assay to determine von Willebrand factor cleaving activity of ADAMTS-13. Thrombosis Res. 118: 723-731.

US 2007/0065895 A1 und US 2005/0186646 A1 offenbaren Verfahren, bei denen eine Probe, die die ADAMTS-13-Protease enthält, mit Peptiden, die eine spezifische Spaltstelle für die ADAMTS-13-Protease aufweisen, beziehungsweise mit VWF-Peptiden (Teilstücken) als Substrat inkubiert wird, und wobei der Nachweis der proteolytischen Aktivität der ADAMTS-13-Protease durch eine anschließende Analyse der resultierenden Peptidfragmente mittels SDS-PAGE und wahlweise anschließendem Immunoblotting erfolgt.

Die Nachteile der bekannten Testsysteme bestehen darin, dass sie entweder technisch sehr aufwändig sind, wie z. B. die Analyse der aus der Proteolyse resultierenden Substratfragmente mittels SDS-PAGE, oder dass sie nicht das natürliche VWF-Substrat verwenden, wodurch manche Dysfunktionalitäten der ADAMTS-13-Protease möglicherweise nicht erkannt werden können.

Der vorliegenden Erfindung lag damit die Aufgabe zugrunde, ein Verfahren zur Bestimmung der von Willebrand Faktor (VWF)-spaltenden Aktivität der ADAMTS-13-Protease bereit zu stellen, das es ermöglicht, so viele Dysfunktionalitäten der ADAMTS-13-Protease wie möglich mit dem Verfahren zu erfassen und das zeitsparend und auf herkömmlichen klinischen Analysegeräten automatisch durchgeführt werden kann.

Die Aufgabe wird dadurch gelöst, dass eine Probe, die vermutlich die ADAMTS-13-Protease enthält, mit isoliertem VWF als Substrat zu einem Reaktionsansatz vermischt wird, wobei das VWF-Substrat aus hochmolekularem, multimerem VWF besteht, welcher VWF-Monomere enthält, die mindestens eine Aminosäuresequenzvariation (Polymorphismus oder Mutation) aufweisen, die jeweils dazu führt, dass das VWF-Substrat -verglichen mit einem VWF-Substrat bestehend aus hochmolekularem, multimerem VWF aus wildtypischen VWF-Monomeren- zu einem beschleunigten Abbau durch ADAMTS-13 führt.

Humanes VWF-Monomer wird in vivo zunächst als 2813 Aminosäuren langes Vorläuferprotein synthetisiert. Durch intrazelluläre Prozessierung entstehen VWF-Multimere, die über 20 000 kDa groß werden können. Diese Multimere bestehen aus linear angeordneten, über Disulfidbrücken untereinander verbundenen 275 kDa großen, 2050 Aminosäuren langen VWF-Monomeren. Im Plasma zirkuliert der VWF in globulärer Form von unterschiedlich großen Multimeren von etwa 500 kDa (Dimer) bis über 15 000 kDa Größe.

Unter einem "hochmolekularen, multimeren VWF-Substrat" ist in Bezug auf die vorliegende Erfindung ein VWF-Protein zu verstehen, dass aus mehr als 10 dimerisierten VWF-Molekülen besteht und dass größer ist als 5000 kDa, was sich durch dem Fachmann bekannte Gelelektrophoreseverfahren feststellen lässt. Weiterhin enthält der hochmolekulare, multimere VWF VWF-Monomere, die mindestens eine Aminosäuresequenzvariation aufweisen, die jeweils dazu führt, dass das VWF-Substrat -verglichen mit einem VWF-Substrat bestehend aus hochmolekularem, multimerem VWF aus wildtypischen VWF-Monomeren- zu einem beschleunigten Abbau durch ADAMTS-13 führt.

Aminosäuresequenzvariationen des VWF-Proteins, die die Empfindlichkeit des VWF für eine proteolytische Spaltung durch die ADAMTS-13-Protease erhöhen, gehören zum Stand der Technik [siehe z. B. Hassenpflug, W.A., Budde, U., Obser, T., Angerhaus, D., Drewke, E., Schneppenheim, S., Schneppenheim, R. (2006) Impact of mutations in the von Willebrand factor A2 domain on ADAMTS13-dependent proteolysis. Blood 107: 2339-2345 oder Rayes, J., Hommais, A., Legendre, P., Tout, H., Veyradier, A., Obert, B., Ribba, A.S., Girma, J.P. (2006) Effect of von Willebrand disease type 2B and type 2M mutations on the susceptibility of von Willebrand factor to ADAMTS-13. J Thromb Haemost. 5: 321-328]. Für die vorliegende Erfindung ist insbesondere ein VWF-Substrat geeignet, das aus hochmolekularem, multimerem VWF besteht, welcher VWF-Monomere enthält, die mindestens eine Aminosäuresequenzvariation aus der folgenden Gruppe aufweisen:

P1648S, E1638K, G1505R, S1506L, M1528V, R1569del, R1597W, V1607D, G1609R, G1629E, G1631D, R1597Q, V1499E und Y1584C.

Die Angabe der Aminosäureposition bezieht sich auf die 2813 Aminosäuren lange Aminosäuresequenz des VWF-Vorläuferproteins (siehe z. B. NCBI Accession No. AAB59458, Version AA59458.1, Gl: 340356). Das Kürzel "dei" bedeutet eine Deletion des betreffenden Aminosäurerests. Zur Nomenklatur von Mutationen und Polymorphismen des von Willebrand Faktors siehe auch Goodeve, A.C., Eikenboorn, J.C.J., Ginsburg, D., Hilbert, L., Mazurier, C., Peake, I.R., Sadler, J.E. and Rodeghiero, F. (2001) A standard nomenclature for von Willebrand factor gene mutations and polymorphisms. Thromb Haemost. 85: 929-931.

Andere natürlich vorkommende oder artifiziell erzeugte Aminosäuresequenzvariationen des VWF können ebenfalls geeignet sein, solange sie jeweils dazu führen, dass das resultierende VWF-Substrat -verglichen mit einem VWF-Substrat bestehend aus hochmolekularem, multimerem VWF aus wildtypischen VWF-Monomeren- zu einem beschleunigten Abbau durch ADAMTS-13 führt, d. h. dass das resultierende VWF-Substrat eine gesteigerte Empfindlichkeit für ADAMTS-13-Proteolyse aufweist. Eine gesteigerte Empfindlichkeit für ADAMTS-13-Proteolyse liegt dann vor, wenn das hochmolekulare, multimere VWF-Substrat in vitro schneller durch ADAMTS-13 proteolytisch abgebaut wird als ein rein wildtypisches hochmolekulares, multimeres VWF-Substrat, d. h. wenn unter gleichen Inkubationsbedingungen eine stärkere Fragmentierung des VWF-Substrats bewirkt wird, die z. B. mitttels eines dem Fachmann bekannten Gelelektrophoreseverfahren festgestellt werden kann.

Das in dem erfindungsgemäßen Verfahren zu verwendende isolierte hochmolekulare, multimere VWF-Substrat kann entweder aus Spenderplasmen von heterozygoten oder homozygoten Trägern einer geeigneten VWF-Aminosäuresequenzvariation gewonnen werden, oder es kann mit Hilfe von dem Fachmann bekannten Verfahren rekombinant exprimiert werden. Rekombinant hergestelltes VWF-Substrat hat die Vorteile, dass es keinerlei ADAMTS-13-Kontamination aufweist, die bei Verschleppung in den Testansatz zu einer Verfälschung der Testergebnisse führen könnte, dass es voll multimerisiert ist und dass es nicht proteolysiert ist, was bei aus Plasma isoliertem VWF der Fall sein kann.

In einer bevorzugten Ausführungsform des erfindungsgemäßen Verfahrens zur Bestimmung der von Willebrand Faktor (VWF)-spaltenden Aktivität der ADAMTS-13-Protease kann der Abbau des VWF-Substrats zusätzlich dadurch beschleunigt werden, dass die Probe zusätzlich mit Heparin vermischt wird.

Die Bestimmung der nach der Inkubation der Probe mit dem VWF-Substrat verbliebenen VWF-Aktivität im Reaktionsansatz erfolgt erfindungsgemäß dadurch, dass der Reaktionsansatz oder ein Aliquot davon mit isoliertem GPlbα-Protein, das eine Aminosäuresequenz umfasst, die verglichen mit der Wildtypsequenz des humanen GPlbα-Proteins (SEQ ID NO:1) mindestens die Aminosäurereste 1-268 enthält und an den Positionen 233 und 239 jeweils eine Aminosäuresubstitution Xaa (bevorzugterweise Xaa = Valin- oder Serin-Rest) aufweist, vermischt wird und dass kein Ristocetin und kein Botrocetin hinzugefügt wird.

Bevorzugterweise bestehen die Substitutionen Xaa des Glycin-Rests an Position 233 und des Methionin-Rests an Position 239 der GPlbα-Kette aus einem ValinRest (G233V bzw. M239V) oder einem Serin-Rest (G233S bzw. M239S). Jede beliebige Kombination der unterschiedlichen Substitutionen Xaa an den beiden Positionen ist möglich.

Bevorzugterweise wird dem Testansatz weiterhin keine "Ristocetin- oder Botrocetin-äquivalente Substanz" hinzugefügt. Unter dem Begriff "Ristocetin- oder Botrocetin-äquivalente Substanz" ist im Sinne der vorliegenden Erfindung eine exogene, d. h. nicht-physiologische Substanz zu verstehen, die in vitro eine Bindung von gelöstem VWF an die wildtypische GPlbα-Kette oder Fragmente davon zu induzieren vermag.

Bei der in dem erfindungsgemäßen Verfahren verwendeten GPlbα-Kette kann es sich um ein rekombinant oder synthetisch hergestelltes GPlbα-Protein handeln. Zur Herstellung von rekombinantem GPlbα-Protein eignen sich bekannte prokaryontische oder eukaryontische Expressionssysteme, wie z. B. die Expression in Bakterien (z. B. E. coli), in Hefen (z. B. Saccharomyces cerevisiae, Pichia pastoris), in pflanzlichen, tierischen oder humanen Zellkulturen. Zur Herstellung von synthetischem GPlbα-Protein eignen sich bekannte Techniken zur in vitro Proteinsynthese, wie z. B. Festphasensynthesen (z. B. Merrifield-Synthese). Bevorzugterweise handelt es sich bei dem in dem erfindungsgemäßen Verfahren verwendeten GPlbα-Protein um rekombinant hergestelltes GPlbα-Protein, das in einer Kultur humaner Zellen, bevorzugt in einer Kultur humaner embryonaler Nierenzellen (HEK-Zellen), hergestellt wurde.

Das in dem erfindungsgemäßen Verfahren verwendete GPlbα-Protein kann am N-Terminus mit der homologen humanen GPlbα-Signalsequenz MPLLLLLLLLPSPLHP (SEQ ID NO: 2, auch als Aminosäurereste -16 bis -1 bezeichnet) fusioniert sein. Alternativ kann das verwendete GPlbα-Protein am N-Terminus mit einer heterologen Signalsequenz fusioniert sein, d. h. mit einem Polypeptid, das üblicherweise nicht in dem humanen GPlbα-Polypeptid vorhanden ist, die aber in dem gewählten Expressionssystem die Expression und/oder Sekretion des rekombinant exprimierten GPlbα-Proteins positiv beeinflusst. Eine geeignete heterologe Signalsequenz ist z. B. MPLQLLLLLILLGPGNSLQLWDTWADEAEKALGPLLARDRR (SEQ ID NO: 3).

Weiterhin kann das in dem erfindungsgemäßen Verfahren verwendete GPlbα-Protein am C-Terminus mit einem oder mehreren Affinitäts-Tags fusioniert sein, die die Bindung des z. B. rekombinant exprimierten Proteins an einen Affinitätsträger ermöglichen, wodurch z. B. die Reinigung von rekombinant exprimiertem GPlbα-Protein ermöglicht wird oder auch die Bindung des rekombinanten GPlbα-Proteins an eine Festphase. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z. B. spezifische Antikörper, immobilisierte Kationen (z. B. Ni²⁺ mit Affinität für His-Tags) oder andere Typen von Bindungspartnem (z. B. Streptavidin mit Affinität für Strep-Tags).

In einer Ausführungsform des erfindungsgemäßen Verfahrens ist das GPlbα-Protein mit einer Festphase assoziiert. Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist das isolierte GPlbα-Protein über eine chemische Bindung an die Festphase gebunden. Ein Beispiel für eine nicht-kovalente Bindung ist die Oberflächenadsorption. Neben einer direkten Bindung an die Festphase kann das isolierte GPlbα-Protein auch indirekt über spezifische Wechselwirkung mit anderen spezifischen Bindungspartnern an die Festphase gebunden sein, z. B. über spezifische Wechselwirkung mit einem Antikörper, bevorzugt mit einem anti-GPlba-Antikörper oder -sofern das isolierte GPlbα-Protein über ein Affinitäts-Tag verfügt- mit einem anti-Affinitäts-Tag-Antikörper.

Der Begriff "Festphase" im Sinne dieser Erfindung beinhaltet einen Gegenstand, der aus porösem und/oder nicht porösem, wasserunlöslichem Material besteht und die unterschiedlichsten Formen aufweisen kann, wie z. B. Gefäß, Röhrchen, Mikrotitrationsplatte (ELISA-Platte), Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z. B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben.

Die Festphase kann einen Überzug aus einer oder mehreren Schichten aufweisen, z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Besonders bevorzugterweise ist das vorgenannte GPlbα-Protein an eine partikuläre Festphase gekoppelt, die in Abhängigkeit von der verbliebenen VWF-Aktivität agglutiniert. Anhand der Agglutinationsreaktion läßt sich die residuale VWF-Aktivität im Reaktionsansatz bestimmen, die wiederum Aufschluss gibt über die in der Probe enthaltene ADAMTS-13-Aktivität.

Eine bevorzugte Ausführungsform des Verfahrens zur Bestimmung der VWF-Aktivität umfasst die Verwendung eines Partikel-assoziierten GPlbα-Proteins, bevorzugt eines Latexpartikel-assoziierten GPlbα-Proteins, und die Bestimmung der VWF-Aktivität anhand der GPlbα-vermittelten Agglutination der partikulären Festphase. Vorzugsweise wird ein über einen Antikörper an die partikuläre Festphase assoziierte GPlbα-Protein verwendet. Zu diesem Zweck sind anti-GPlbα-Antikörper geeignet, insbesondere die monoklonalen Antikörper VM16d [Mazurov, A.V. et al. (1991) Characterization of an antiglycoprotein Ib monoclonals antibody that specifically inhibits platelet-thrombin interaction. Thromb Res. 62(6), 673-684; kommerziell erhältlich z. B. von Sanbio B.V., Uden, Niederlande: Produktnummer MON 1146] und SZ-2 [Ruan, C. et al. (1987) A murine antiglycoprotein Ib complex monoclonal antibody, SZ 2, inhibits platelet aggregation induced by both ristocetin and collagen. Blood 69(2), 570-577; kommerziell erhältlich z. B. von Beckman Coulter Inc., Fullerton, USA: Produktnummer IM0409]. Sofern das verwendete GPlbα-Protein am C-Terminus mit einem oder mehreren Flag-Tags fusioniert ist, ist gleichermaßen ein anti-Flag-Antikörper geeignet [siehe z. B. US 5,011,912; kommerziell erhältlich z. B. von Sigma-Aldrich Chemie GmbH, Steinheim, Deutschland]. Zur quantitativen Bestimmung der Agglutinationsreaktion, die mit der Menge bzw. Aktivität des in der Probe vorhandenen VWF korreliert, kann z. B. die Lichtstreuung an den Partikelaggregaten über die Messung der Streulichintensität (Nephelometrie) oder über die Messung der Trübung des Mediums (Turbidimetrie) genutzt werden.

Bevorzugterweise wird die Bestimmung der VWF-Aktivität anhand der GPlbα-vermittelten Agglutination einer partikulären Festphase in Gegenwart eines Detergenzes durchgeführt, bevorzugterweise in Gegenwart eines Detergenzes aus der Gruppe Tween® 20, Thesit, Triton-Detergenz (z. B. Triton X-100® oder Triton X-405®) und Natriumdodecylsulfat (SDS). Es wurde gefunden, dass die Gegenwart eines Detergenzes die VWF-abhängige Agglutinationsrate der partikulären Festphase, insbesondere von Latexpartikeln, beeinflusst:

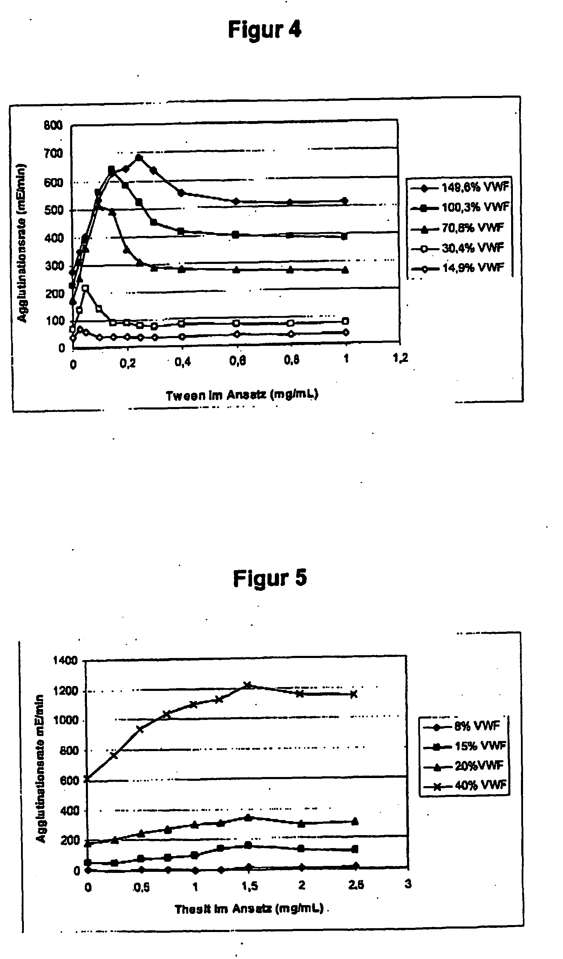

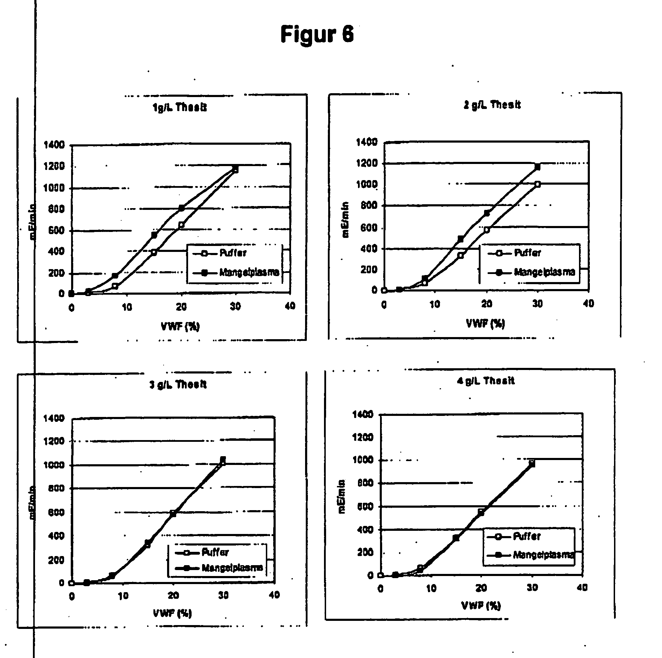

Bei verschiedenen VWF-Konzentrationen und bei steigenden Tween® 20-Konzentrationen wurde die Agglutinationsrate bestimmt (siehe Figur 4, Bestimmung analog Beispiel 5). Es wird deutlich, dass es bei relativ geringen Tween® 20-Konzentrationen (0,02-0,1 g/L) eine besonders ausgeprägte Verstärkung der Reaktion gibt, welche mit steigender Tween® 20-Konzentration wieder geringer wird und ein stabiles Niveau an Verstärkung erreicht. So ist z. B. bei 149,6 % VWF bei ca. 1 g/L Tween® 20 das stabile Niveau der Verstärkung erreicht, und die Verstärkung beträgt dann 85 % vom Ausgangswert ohne Tween® 20. Wenn Tween® 20 als Detergenz benutzt wird, bestehen folglich zwei Möglichkeiten. Zum einen kann Tween® 20 bei geringen Konzentrationen (0,02-0,1 g/L) eingestellt werden, um bestimmte VWF-Konzentrationen bis zu 20 % besonders stark zu verstärken. So erhält man z. B. bei 0,05 g/L Tween® 20 (im Testansatz) eine Verstärkung der Agglutinationsrate um 216 % bei 30,4 % VWF. Da niedrige VWF-Konzentrationen besonders schwierig zu bestimmen sind, ist hier eine Verstärkung der Reaktion sehr hilfreich. Zum anderen ist ein Meßsystem generell stabiler, wenn im Sättigungsbereich des Detergenzes für alle relevanten VWF-Konzentrationen gearbeitet wird. Diese wäre z. B. ab 1 g/L Tween® 20 der Fall. Diese Variante ist besser für einen Screening-Test für ein weites Spektrum an VWF-Konzentrationen geeignet. Tween® 20-Konzentrationen von 0,6-20,0 g/L, bevorzugt von 1 g/L, sind vorteilhaft, wenn die Agglutinationsreaktion bei der Bestimmung von VWF-Konzentrationen von mehr als 20 % verstärkt werden soll.

Andere Detergenzien, wie Thesit (synonym: Polydocanol, Schärer & Schläpfer Ltd., Rothrist, Schweiz), Triton-Detergenz (z. B. Triton X-100® oder Triton X-405®) und Natriumdodecylsulfat (SDS) zeigen einen stetigen konzentrationsabhängigen Anstieg der Verstärkung der Agglutinationsreaktion (siehe Figur 5, Bestimmung analog Beispiel 5: 45 µL Probe jedoch mit Thesit anstelle von Tween® 20).

Weiterhin bevorzugt ist die Bestimmung der VWF-Aktivität anhand der GPlbα-vermittelten Agglutination einer partikulären Festphase in Gegenwart von Polyvinylpyrrolidon (PVP), bevorzugterweise bei einer Polyvinylpyrrolidon-Konzentration von 0,1 % bis 1,0 % im Testansatz und/oder in Gegenwart von Dextran T500, bevorzugterweise bei einer Dextran T500-Konzentration von 0,1 % bis 3 % im Testansatz und/oder in Gegenwart von Alginate 500-600 cP, bevorzugterweise bei einer Alginate 500-600 cP-Konzentration von 0,01 % bis 0,2 % im Testansatz.

Bei einer anderen bevorzugten Ausführungsform des erfindungsgemäßen Verfahrens zur Bestimmung der VWF-Aktivität handelt es sich um ein kompetitives Testformat. Das Verfahren umfasst die Verwendung von Partikel-assoziiertem anti-GPlbα-Antikörper, bevorzugt VM16d, und von Partikel-assoziiertem VWF in einem Reaktionsansatz und die Zugabe von GPlbα-Protein. In Gegenwart des GPlbα-Proteins und in Abwesenheit von Ristocetin, Botrocetin oder einer äquivalenten Substanz agglutinieren die Partikel. Die Zugabe einer VWFenthaltenden Probe hemmt diese Agglutinationsreaktion. Die Hemmung der Agglutinationsreaktion korreliert mit der Menge bzw. Aktivität des in der Probe vorhandenen VWF.

Eine andere bevorzugte Ausführungsform des Verfahrens zur Bestimmung der VWF-Aktivität umfasst die Verwendung eines an eine nicht-partikuläre Festphase assoziierten, bevorzugterweise eines an die Oberfläche einer Mikrotiterplatte assoziierten GPlbα-Proteins und die Bestimmung der VWF-Aktivität anhand der Bestimmung der VWF-Menge, die an das GPlbα-Protein gebunden wurde. Vorzugsweise wird ein über einen Antikörper an die nicht-partikuläre Festphase assoziiertes GPlbα-Protein verwendet. Zu diesem Zweck sind anti-GPlbα-Antikörper geeignet, insbesondere die monoklonalen Antikörper VM16d (siehe oben) und MB45 [siehe z. B. Modderman, P.W. et al. (1992) Glycoproteins V and Ib-IX form a noncovalent complex in the platelet membrane. J. Biol. Chem. 267(1), 364-369 und darin zitierte Referenz 26; kommerziell erhältlich z. B. von Sanquin, Amsterdam Niederlande: PeliCluster CD42b, Produktnummer M9142]. Sofern das verwendete GPlbα-Protein am C-Terminus mit einem oder mehreren Flag-Tags fusioniert ist, ist gleichermaßen ein anti-Flag-Antikörper geeignet [siehe oben]. Zur Bestimmung der VWF-Menge, die an das GPlbα-Protein gebunden wurde kann beispielweise ein anti-VWF-Antikörper verwendet werden, der direkt oder indirekt mit einer Komponente eines signalbildenden Systems assoziiert ist, und damit die Quantifizierung der GPlbα-gebundenen VWF-Menge erlaubt. Da nur "aktiver" (funktionell intakter) VWF an den GPlbα-Rezeptor bindet, korrelliert die nach diesem Prinzip ermittelte VWF-Antigenkonzentration mit der VWF-Aktivität.

Ein besonderer Vorteil des erfindungsgemäßen Verfahrens zur Bestimmung der Willebrand Faktor (VWF)-spaltenden Aktivität der ADAMTS-13-Protease in einer Probe besteht darin, dass auf eine Harnstoff-Behandlung des VWF-Substrats verzichtet werden kann, deren Anwendung mit dem Risiko verbunden ist, dass anschließend zu niedrige VWF-Aktivitäten bestimmt werden, wenn die Inkubationszeiten bei der Vorbehandlung nicht strikt eingehalten werden. Bei diversen Verfahren aus dem Stand der Technik ist die Vorbehandlung des VWF-Substrats mit Harnstoff oder ähnlich denaturierend wirkenden Substanzen notwendig, um den VWF für den Abbau durch die ADAMTS-13-Protease überhaupt zugänglich zu machen (siehe z. B. WO 2004/005451 A2).

Da in jeder humanen Plasmaprobe, in der die VWF-spaltende Aktivität der ADAMTS-13-Protease bestimmt werden soll, probenintrinsischer VWF enthalten ist, ist es vorteilhaft, parallel zum eigentlichen Testansatz eine Messung eines zweiten Testansatzes durchzuführen, bei der ein zweites Aliquot derselben Probe mit Hilfe des erfindungsgemäßen Verfahrens analysiert wird, allerdings ohne dass die Probe, die mit dem VWF-Substrat vermischt ist, über denselben Zeitraum inkubiert wird wie der eigentliche Testansatz, sondern wobei unmittelbar nach dem Vermischen von Probe und VWF-Substrat die VWF-Aktivität im zweiten Testansatz gemessen wird. Die Differenz zwischen der VWF-Aktivität in dem zweiten Testansatz (ohne Inkubationsschritt) und der VWF-Aktivität im eigentlichen Testansatz (mit Inkubationsschritt zum proteolytischen VWF-Abbau) ergibt dann die im eigentlichen Testansatz durch ADAMTS-13 abgebaute VWF-Aktivität.

### Figurenbeschreibung

**Figur 1**
   Der Aufbau der hier angewendeten GPlbα-(aa1-285,G233V/M239V)- bzw. Wildtyp-GPlbα-ELISA Testsysteme zur Bestimmung von VWF ist in Beispiel 4 angegeben. Es wurden 4 verschiedene Verdünnungen des VWF/Faktor VIII-Konzentrats Haemate® als Probe eingesetzt. In Abwesenheit von Ristocetin (0 mg/mL) erfolgt bei Verwendung des mutanten GPlbα-(aa1-285,G233V/M239V) eine starke und konzentrationsabhängige Bindung von VWF (Abbildung oben). Durch Zugabe von Ristocetin ist diese Bindung geringfügig steigerbar, was wahrscheinlich auf eine unspezifische Bindung zurückzuführen ist, die auch ohne GPlbα stattfindet. Hingegen ist eine ähnlich starke VWF-Bindung beim wildtypischen GPlbα nur durch Zugabe von 1,2 mg/mL Ristocetin zu erreichen. Ohne Ristocetinzugabe findet bei Verwendung von wildtypischem GPlbα überhaupt keine Bindung statt (Abbildung unten).
**Figur 2**
   Kalibrationskurve zur Bestimmung der VWF-Aktivität (10 % - 200 % VWF-Aktivität) in humanen Citratplasmaproben mittels eines GPlbα-Latexpartikel-Agglutinationstests ohne Ristocetin (siehe Beispiel 5). Darstellung der Reaktionsgeschwindigkeit der Agglutination der Partikel in Abhängigkeit von der VWF-Aktivität.
**Figur 3**
   Kalibrationskurve zur Bestimmung niedriger VWF-Aktivitäten (≥ 3 % VWF-Aktivität) in humanen Citratplasmaproben mittels eines GPlbα-Latexpartikel-Agglutinationstests ohne Ristocetin (siehe Beispiel 5, 45 µL Probe). Darstellung der Reaktionsgeschwindigkeit der Agglutination der Partikel in Abhängigkeit von der VWF-Aktivität.
**Figur 4**
   GPlbα-Latexpartikel-Agglutinationstests ohne Ristocetin in Proben mit unterschiedlicher VWF-Konzentration (14,9 % bis 149,6 %) und in Gegenwart unterschiedlicher Tween® 20-Konzentrationen im Testansatz (siehe Beispiel 5, Variation der Tween® 20-Konzentration, 15 µL Probe).
**Figur 5**
   GPlbα-Latexpartikel-Agglutinationstests ohne Ristocetin in Proben mit unterschiedlicher VWF-Konzentration (8 % bis 40 %) und in Gegenwart unterschiedlicher Thesit-Konzentrationen im Testansatz (siehe Beispiel 5, jedoch Thesit anstelle von Tween® 20, 45 µL Probe).
**Figur 6**
   GPlbα-Latexpartikel-Agglutinationstests ohne Ristocetin in Proben mit unterschiedlicher VWF-Konzentration, die mit VWF-Mangelplasma bzw. mit Puffer vorverdünnt wurden, und in Gegenwart unterschiedlicher Thesit-Konzentrationen im Testansatz (siehe Beispiel 5, jedoch Thesit anstelle von Tween® 20, 45 µL Probe).

Die im Folgenden beschriebenen Beispiele dienen der exemplarischen Beleuchtung einzelner Aspekte dieser Erfindung und sind nicht als Einschränkung zu verstehen.

### Beispiele

### Beispiel 1: Klonierung des GPlbα-(G233V/M239V)-(3×Flag)-(6×His)-Konstrukts

Der Expressionsvektor pIRES neo2 (BD Biosciences, Clontech, Art.-Nr.: 6938-1) wurde so modifiziert, dass er zwischen der EcoRI- und der Notl-Restriktionsschnittstelle ein 3×Flag-Tag und ein 6xHis-Tag enthielt. Stromaufwärts (5') der Tag-Sequenzen wurde ein für das Signalpeptid (SEQ ID NO: 2) und die Aminosäuren 1-285 des humanen GPlbα-Rezeptors (SEQ ID NO: 1) codierendes Fragment, das an den für die Aminosäurepositionen 233 und 239 codierenden Stellen jeweils ein für Valin codierendes Codon enthielt, inseriert.

### Beispiel 2: Expression des rekombinanten GPlbα-(G233V/M239V)-(3×Flag)-(6×His)-Fusionsproteins in HEK-Zellen

HEK 293 Zellen (humane embryonale Nierenzellen; ATCC-Number: CRL-1573; Cell Lines Service CLS, Heidelberg) wurden mit dem in Beispiel 1 beschriebenen Konstrukt transformiert.

Die Zellen werden kultiviert in:
DMEM (Art.-Nr.:31966-021, Invitrogen)
+ 10% FBS Origin EU (Art.-Nr.:10270-106, Invitrogen), das hitzeinaktiviert wurde oder aber in 10% FBS Origin USA (Art.-Nr.:A15-003, Lot-No.:A01123-678, PAA)
+ 1 % Antibiotic-Antimycotic-Solution(100×) (Art.-Nr.:P11-002, PAA)
+ 0,1% Gentamycin-Solution 50mg/ml (Art.-Nr.:P11-005, PAA)
+ 500µg/ml Geneticin (G418)-Solution 50mg/ml active Geneticin (Art.-Nr.:10131-027, Invitrogen)

Für die Expression (Produktion):
OPTIPRO-SFM (Art.-Nr.:12309-019, Invitrogen)
+ 0,5% Antibiotic-Antimycotic-Solution(100×) (Art.-Nr.:P11-002, PAA)
+ 0,05% Gentamycin-Solution 50mg/ml (Art.-Nr.:P11-005, PAA)
+ 2% Glutamax I (100×) (Art.-Nr.: 35050-038, Invitrogen)
und kein Geneticin

Der Ablauf ist wie folgt:
1) Auftauen der Zellen (1 Kryo mit 5-10x 10e6 Zellen) und Kultivierung in T175 in serumhaltigem Medium für 96h
2) Splitten auf 4x T175 und Kultivierung für 72-96h
3) Splitten auf 25x T175 und Kultivierung für 72-96h
4) Eine T175 splitten und als Reserve weiter kultivieren. Die restlichen 24x T175 splitten auf 3x CellStack Corning (je CellStack 10 Ebenen mit insgesamt 6360cm2) und für 72h kultivieren
5) Medium entfernen Monolayer mit DMEM ohne FBS 1-2x waschen und serumfreies OPTIPRO-SFM (1,8 Liter je CellStack) zugeben und für 96h kultivieren
6) Ernte des Mediums. Überstand abzentrifugieren und Pellet der abgelösten Zellen wiederaufnehmen und zurück in den CellStack mit frischem OPTIPRO-SFM geben und für 96h kultivieren
7) wie unter 6)
8) Finale Ernte des Mediums und Beendung der Kultur

### Beispiel 3: Affinitätschromatographische Isolierung des rekombinanten GPlbα-(G233V/M239V)-(3×Flag)-(6×His)-Fusionsproteins

Das nach Beispiel 2 gewonnene GPlbα-(G233V/M239V)-(3×Flag)-(6×His) enthaltende Medium wird durch Zentrifugation (35 min, 10.000 UpM, Beckman J2-21, Beckman Coulter GmbH, Deutschland) von noch vorhanden Zellen oder Zellbruchstücken befreit. Der so erhaltene zellfreie Überstand wird mittels Tangentialultrafiltration unter Verwendung einer Ultraflitrationskassette mit einer Trenngrenze von 10 kDa (PES 10, Schleicher & Schüll, Deutschland) auf 1/10 des Ausgangsvolumens ankonzentriert.

Die Reinigung erfolgt mittels Affinitätschromatographie unter Verwendung einer Ni²⁺-Sepharose (His Prep FF16/10, GE Healthcare, Schweden) nach Angaben des Herstellers. Zur Bindung des GPlbα-(G233V/M239V)-(3×Flag)-(6×His) werden dem konzentrierten Überstand 500 mmol NaCl, 20 mmol Na₂HPO₄ und 5 mM Imidazol zugegeben und durch Zugabe von 5 M HCl der pH-Wert auf pH 7,4 eingestellt. Nichtgebundene Komponenten werden durch Spülen der Säule mit einem Puffer aus 500 mMol NaCl, 20 mmol Na₂HPO₄, 5 mM Imidazol, pH 7,4 abgewaschen. Die Eluation des gebundenen GPlbα-(G233V/M239V)-(3×Flag)-(6xHis) erfolgt mit einem Puffer aus 20 mMol Na₂HPO₄, 500 mMol NaCl, 500 mMol Imidazol, pH 7,4. Das so erhaltene Eluat wird in einer Ultrafiltrationsrührzelle mit einer Ultrafiltrationsmembran, Trenngrenze 10 kDa (OMEGA 10 K, Pall Life Sciences, USA) auf 1/10 des Ausgangsvolumens ankonzentriert. Die weitere Reinigung und Abtrennung von Kontaminationen erfolgt mittels Gelfitration unter Verwendung einer Chromatographiesäule Superdex 200 prep grade 35/600 (GE Healthcare, Schweden) nach Angaben des Herstellers. Die Chromatographie erfolgt mit einer Flussrate von 5,0 ml/min unter Verwendung eines Puffers aus 0,048 mol/L Na₂HPO₄, 0,02 mol/L KH₂PO₄, 0,145 mol/L NaCl, 0,015 mol/L NaN₃, pH 7,2. Nach Auftragen der Probe eluiert GPlbα-(G233V/M239V)-(3×Flag)-(6xHis) in einem Peak nach einem Eluationsvolumen von ca. 300 ml von der eingesetzten Chromatographiesäule.

### Beispiel 4: Verfahren zur Bestimmung der VWF-Aktivität in einem Anti-FLAG/GPIba-ELISA ohne Ristocetin

Es wurden ELISA-Platten benutzt, die bereits mit einem Antikörper gegen das Flag-Tag beschichtet sind (Sigma, Saint Louis, USA, ANTI-FLAG HS, M2 coated 96-well Plates (clear), Product Number P 2983). In jede Vertiefung der ELISA-Platte wurden 100 µL einer Phosphatpufferlösung gegeben, die rekombinantes wildtypisches GPlba-(3×Flag)-(6×His)-Fusionsprotein (1:10 verdünnter Überstand des Kulturmediums nach Zellsedimentation) oder isoliertes rekombinantes GPlbα-(aa1-285,G233V/M239V)-(3×Flag)-(6×His)-Fusionsprotein (siehe Beispiel 3) in einer Konzentration von 2,4 µg/mL enthielt, und es wurde über Nacht bei 2-8 °C inkubiert. Nach 4-fachem Waschen mit Phosphatpuffer (+ 0,01 % Tween®) wurden 50 µL einer Verdünnung von Hämate® (ZLB Behring, Marburg, Deutschland) in Phosphatpuffer + 0,1 % Rinderserumalbumin sowie 50 µL einer Ristocetin-Verdünnung in Phosphatpuffer + 0,1% Rinderserumalbumin zugegeben. Danach erfolgte eine Inkubation für 1 Stunde bei Raumtemperatur. Nach 4-fachem Waschen wie oben wurden 100 µL eines horse raddish Peroxidase-gekoppelten anti-VWF-Antikörpers (Rabbit Anti-Human VWF/HRP, DAKO, Ref. P0226) zugegeben, 1 Stunde bei Raumtemperatur inkubiert und danach wie oben gewaschen. Daraufhin wurden 100 µL Tetramethylbenzidin-Lösung als Substrat (TMB Substrat, Dade Behring Marburg GmbH, Marburg, Deutschland, Ref. 450684) zugegeben, 4 Minuten bei Raumtemperatur inkubiert. Durch Zugabe von 100 µL 0,5 N Schwefelsäure wurde die Reaktion abgestoppt. Danach erfolgte die Messung der Extinktion bei 405 nm in einem Spektralphotometer.

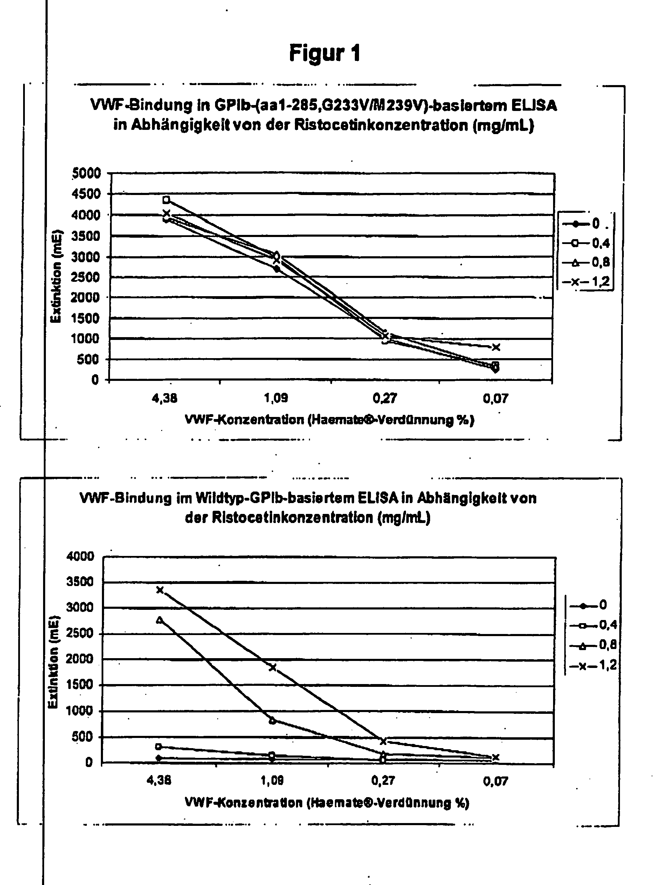

Bei Abwesenheit von Ristocetin erfolgte bei Verwendung des gemäß den Beispielen 1-3 hergestellten mutanten GPlbα eine starke und konzentrationsabhängige Bindung von VWF. Durch Zugabe von Ristocetin wurde diese Bindung geringfügig gesteigert, was wahrscheinlich auf eine unspezifische Bindung zurückzuführen ist, die auch ohne GPlbα stattfindet. Hingegen ist eine ähnlich starke VWF-Bindung beim wildtypischen GPlbα nur durch Zugabe von 1,2 mg/mL Ristocetin zu erreichen. Ohne Ristocetinzugabe findet bei Verwendung von wildtypischem GPlbα überhaupt keine Bindung statt (Figur 1).

### Beispiel 5: Verfahren zur Bestimmung der VWF-Aktivität in einem GPlbα-Latexpartikel-Agglutinationstest ohne Ristocetin

Es wurden drei Reagenzien vorbereitet:

### Reagenz 1:

Der monoklonale anti-GPlbα-Antikörper VM16d (Sanbio B.V., Uden, Niederlande: Produktnummer MON 1146) wurde auf der Oberfläche von Latexpartikeln gebunden, und die Partikel in einem Puffer zur Langzeitlagerung resuspendiert (0,6 g/L TRIS, 1,1 % Leucin, 12,5 % Saccharose, 0,05 % HSA, 6,25 mg/L Gentamycin und 0,625 mg/L Amphotericin, pH 8.25, Latexkonzentration 0,22 %).

### Reagenz 2:

### VWF-Mangelplasma

### Reagenz 3:

Die folgenden Komponenten wurden gemischt und ergaben ein Gesamtvolumen von 30 mL Reagenz 3:
2,5 mL einer 7,5 %igen Lösung von Polyvinylpyrrolidon (PVP) (Fluka, Deutschland) in Phosphatpuffer (pH 7,1),
12,9 mL (0,625 mg/mL) Heterophilic Blocking Reagent 1 (Scantibodies Laboratory Inc, Santee, CA 92071, USA) in Tris-Puffer (pH 7,1),
3,4 mL einer Lösung mit 21 g/L Tween® 20 (Sigma, St. Louis, Missouri, USA) in Phosphatpuffer (pH 7,2),
0,086 mL einer Lösung von 5,59 mg/mL rekombinantem GPlbα-(G233V/M239V)-(3×Flag)-(6×His) (siehe Beispiel 3) in Phosphatpuffer (pH 7,1), und
11,2 mL Phosphatpuffer (pH 7,1).

Die Testdurchführung erfolgte an dem automatischem Gerinnungsanalysengerät BCS® (Behring Coagulation System, Dade Behring Marburg GmbH, Marburg, Deutschland). Es wurden 15 µL einer humanen Citratplasma-Probe, 30 µL Reagenz 2 und 70 µL Reagenz 3 gemischt. Der Start der Reaktion erfolgte durch Zugabe und Mischung mit 40 µL Reagenz 1. Die Extinktion der Reaktionsmischung wurde kontinuierlich bei 575 nm gemessen. Die Geschwindigkeit der Agglutination der Latexpartikel erfolgte in Abhängigkeit von der Aktivität des VWF in der Probe.

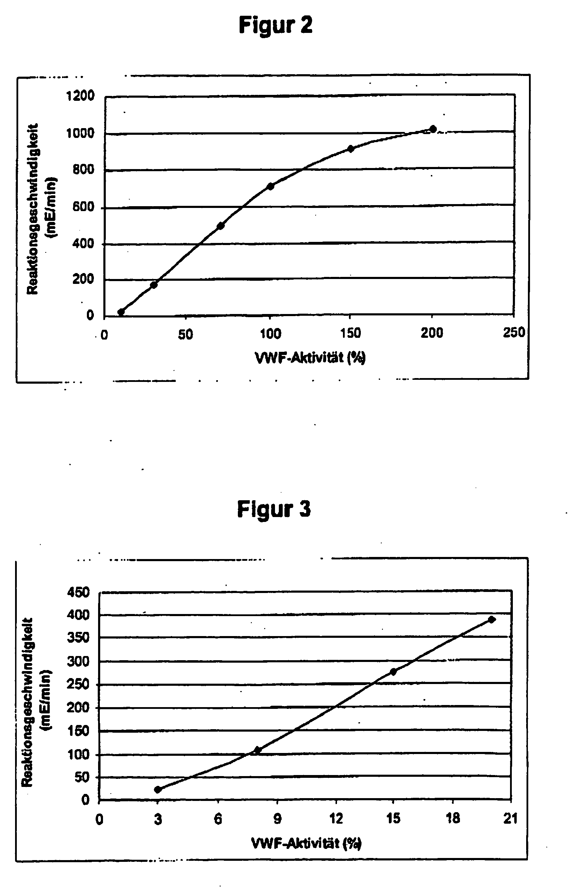

Zur Berechnung der VWF-Aktivität wurde eine Kalibrationskurve unter Benutzung von Standard Human Plasma (Dade Behring Marburg GmbH, Marburg, Deutschland) erstellt. Als VWF-Sollwert wurde der deklarierte Ristocetin-Kofaktor-Wert für das BCS®-System verwendet. Durch Erhöhung des Volumens von Standard Human Plasma bei gleichzeitiger Reduktion der entsprechende Menge an Reagenz 2 wurden VWF-Aktivitäten bis zu 200 % erzeugt. Durch Verdünnung des Standard Human Plasmas mit Reagenz 2 wurden niedrigere VWF-Aktivitäten erzeugt. Eine typische Kalibrationskurve ist in Figur 2 dargestellt. Die VWF-Aktivität einer Probe kann mit Hilfe der ermittelten Reaktionsgeschwindigkeit an der Kalibrationskurve abgelesen werden.

In einem Testansatz zur Messung besonders niedriger VWF-Aktivitäten in einer Probe wurden anstelle von 15 bzw. 30 µL Probe (siehe oben) 45 µL Probe eingesetzt. Bei der Kaübration wurde Standard Human Plasma mit Reagenz 2 so verdünnt, dass niedrige VWF-Aktivitäten von bis zu 3 % erzeugt wurden (siehe Figur 3). Dieser Testansatz ermöglicht die genaue Bestimmung extrem niedriger VWF-Aktivitäten, was besonders wichtig bei der Bildung von VWF-Aktivität/Antigen-Ratios ist, um Subtypen des Von Willebrand-Syndroms zu klassifizieren. Durch ein Erhöhung der PVP-Konzentration auf 0,35 % im Ansatz und des Probenvolumens auf 60 µL kann die VWF-abhängige Agglutination zusätzlich verstärkt werden und es kann noch eine Differenzierung von 1 % VWF und 0 % VWF erfolgen. Somit kann mit großer Sicherheit eine Typ 3-Von Willebrand Erkrankung (0 % VWF) von extrem starken Defizienzen anderer Typen (z. B. 1-5 %) unterschieden werden.

### Beispiel 6: Erfindungsgemäßes Verfahren zur Bestimmung der VWF-spaltenden Aktivität der ADAMTS-13-Protease mit Harnstoff-vorbehandeltem, hochmolekularem, multimerem VWF (P1648S) als VWF-Substrat

1. Reaktionspuffer: 1 mmol/L Pefabloc SC (Roche Diagnostics GmbH, Mannheim, Germany), 12,5 mmol/L BaCl₂, 5 mmol/L TRIS, pH 8,0;
2. Substratpuffer ansetzen und exakt 5 Minuten bei Raumtemperatur inkubieren: 8,3 U/mL (830 %) hochmolekularen, multimeren, rekombinant hergestelltem VWF-P1648S, 4,6 mol/L Harnstoff, 4,6 mmol/L TRIS, pH 8,0;
3. 15 µL humane Plasmaprobe werden mit 300 µL Reaktionspuffer versetzt und anschließend mit 150 µL Substratpuffer vermischt;
4. Inkubation des Testansatzes für 4 bis 8 Stunden bei 37 °C;
5. Messung der VWF-Aktivität unter Einsatz dieses Reaktionsgemisches als Probe wie in Beispiel 5 beschrieben.

### Beispiel 7: Erfindungsgemäßes Verfahren zur Bestimmung der VWF-spaltenden Aktivität der ADAMTS-13-Protease mit Harnstoff-vorbehandeltem, rekombinantem hochmolekularem, multimerem VWF (P1648S) als VWF-Substrat

1. 6 µL humane Plasmaprobe (1:6 verdünnt mit Reaktionspuffer, siehe Beispiel 6) werden mit 20 µL Reaktionspuffer vermischt;
2. Inkubation des Gemisches für 5 Minuten bei 37 °C zur Aktivierung der ADAMTS-13-Protease;
3. Zugabe von 12 µL Substratpuffer (5 U/mL (500 %) rekombinanten hochmolekularen, multimeren VWF-P1648S, 5 mol/L Harnstoff, 5 mmol/L Tris-HCl, pH 8,0);
4. Inkubation des Testansatzes für 20 Minuten bei 37 °C;
5. Messung der VWF-Aktivität unter Einsatz dieses Reaktionsgemisches als Probe wie in Beispiel 5 beschrieben.

### Beispiel 8: Erfindungsgemäßes Verfahren zur Bestimmung der VWF-spaltenden Aktivität der ADAMTS-13-Protease mit hochmolekularem, multimerem VWF (P1648S) als VWF-Substrat und Differenzbildung mit/ohne ADAMTS-13 Abbau

### Test 1:

1. 10 µL einer humanen Plasmaprobe werden mit 20 µL ADAMTS-13 Aktivierungspuffer (18,75 mmol/L BaCl₂, pH 8,0, 5 mmol/L Tris-HCl, 1,5 mmol/L Pefabloc SC (Roche Diagnostics GmbH, Mannheim, Germany)) vermischt;
2. Inkubation des Gemisches für 5 Minuten bei 37 °C;
3. Zugabe von 20 µL mit 1,25 U/mL (125 %) rekombinantem VWF-Substrat (P1648S) in 5 mmol/L Tris-HCl, pH 8,0;
4. Inkubation des Testansatzes für 20 Minuten bei 37 °C;
5. Messung der VWF-Aktivität unter Einsatz dieses Reaktionsgemisches als Probe wie in Beispiel 5 beschrieben.

### Test 2:

Weitere 10 µL derselben humanen Plasmaprobe werden wie in Test 1 behandelt, allerdings mit dem Unterschied, dass der Inkubationsschritt für 20 Minuten bei 37 °C (siehe Schritt Nr. 4) nicht durchgeführt wird, sondern die Messung der VWF-Aktivität unter Einsatz dieses Reaktionsgemisches als Probe wie in Beispiel 5 unmittelbar nach der Zugabe des VWF-Substrats (P1648S) erfolgt. Die Differenz der VWF-Aktivität von Test 1 und Test 2 ergibt die abgebaute Menge an VWF-Aktivität und damit die ADAMTS-13-Aktivität. Anhand einer Kalibration wird daraus die Aktivität von ADAMTS-13 in der Plasmaprobe bestimmt.

## Patentansprüche

1. Verfahren zur Bestimmung der von Willebrand Faktor (VWF)-spaltenden Aktivität der ADAMTS-13-Protease In einer Probe, umfassend folgende Schritte:
a) Vermischen der Probe, die vermutlich die ADAMTS-13-Protease enthält, mit isoliertem VWF als Substrat zu einem Reaktionsansatz,
b) Inkubation des Reaktionsansatzes, und
c) Bestimmung der verbliebenen VWF-Aktivität im Reaktionsansatz, wobei
das VWF-Substrat aus hochmolekularem, multimerem VWF besteht, welcher VWF-Monomere enthält, die mindestens eine Aminosäuresequenzvariation aufweisen, die dazu führt, dass das VWF-Substrat -verglichen mit einem VWF-Substrat bestehend aus hochmolekularem, multimerem VWF aus wildtypischen VWF-Monomerenzu einem beschleunigten Abbau durch ADAMTS-13 führt,
**dadurch gekennzeichnet, dass**
in Schritt c) die Bestimmung der verbliebenen VWF-Aktivität im Reaktionsansatz mit einem Verfahren erfolgt, bei welchem die Probe mit isoliertem GPlbα-Protein, das eine Aminosäuresequenz umfasst, die verglichen mit der Wildtypsequenz des humanen GPlbα-Proteins (SEQ ID NO:1) mindestens die Aminosäurereste 1-268 enthält und an den Positionen 233 und 239 jeweils eine Aminosäuresubstitution Xaa aufweist, zu einem Testansatz vermischt wird, und bei welchem dem Testansatz kein Ristocetin und kein Botrocetin hinzugefügt wird.

2. Verfahren gemäß Anspruch 1, wobei VWF-Monomere des VWF-Substrates aus hochmolekularem, multimerem VWF mindestens eine Aminosäuresequenzvariation aus der folgenden Gruppe aufweisen:
P1648S, E1638K, G1505R, S1506L, M1528V, R1569del, R1597W, V1607D, G1609R, G1629E, G1631D, R1597Q, V1499E und Y1584C.

3. Verfahren gemäß einem der Ansprüche 1 oder 2, wobei das hochmolekulare, multimere VWF-Substrat rekombinant hergestellt ist.

4. Verfahren gemäß einem der Ansprüche 1 oder 2, wobei das hochmolekulare, multimere VWF-Substrat aus Spenderplasmen von heterozygoten oder homozygoten Trägern einer geeigneten VWF-Aminosäuresequenzvariation isoliert ist.

5. Verfahren nach einem der vorhergehenden Ansprüche, wobei in Schritt a) die Probe zusätzlich mit Heparin vermischt wird.

6. Verfahren nach einem der vorhergehenden Ansprüche, **dadurch gekennzeichnet, dass** die Aminosäuresubstitution Xaa an Position 233 und/oder Position 239 des verwendeten GPlbα-Proteins aus einem Valin- oder einem Serin-Rest besteht.

7. Verfahren nach einem der vorhergehenden Ansprüche, **dadurch gekennzeichnet, dass** das verwendete GPlbα-Protein mit einer Festphase, bevorzugt mit einer partikulären Festphase, assoziiert ist.

8. Verfahren nach Anspruch 7, **dadurch gekennzeichnet, dass** die VWF-Aktivität anhand der GPlbα-vermittelten Agglutination der partikulären Festphase bestimmt wird.

## Claims

1. A method for determining the von Willebrand factor (VWF)-cleaving activity of ADAMTS-13 protease in a sample, comprising the following steps:
a) mixing the sample which is suspected of containing the ADAMTS-13 protease with isolated VWF as substrate to give a reaction mix,
b) incubating the reaction mix, and
c) determining the remaining VWF activity in the reaction mix,
wherein
the VWF substrate is high molecular weight, multimeric VWF which includes VWF monomers having at least one amino acid sequence variation resulting in the VWF substrate being broken down by ADAMTS-13 in an accelerated manner compared to a VWF substrate that is high molecular weight, multimeric VWF composed of wild-type VWF monomers,
**characterized in that**
step c) comprises determining the remaining VWF activity in the reaction mix using a method in which the sample is mixed with isolated GPIbα protein comprising an amino acid sequence which, when compared to the wild-type sequence of human GPIbα protein (SEQ ID NO: 1), includes at least the amino acid residues 1-268 and has in each case one amino acid substitution Xaa in positions 233 and 239, to give an assay mix, and in which there is added to the assay mix neither ristocetin nor botrocetin.

2. The method as claimed in claim 1, wherein VWF monomers of the VWF substrate composed of high molecular weight, multimeric VWF have at least one amino acid sequence variation from the following group:
P1648S, E1638K, G1505R, S1506L, M1528V, R1569del, R1597W, V1607D, G1609R, G1629E, G1631D, R1597Q, V1499E and Y1584C.

3. The method as claimed in either of claims 1 and 2, wherein the high molecular weight, multimeric VWF substrate is produced in a recombinant manner.

4. The method as claimed in either of claims 1 and 2, wherein the high molecular weight, multimeric VWF substrate has been isolated from donor plasmas of heterozygous or homozygous carriers of a suitable VWF amino acid sequence variation.

5. The method as claimed in any of the preceding claims, wherein step a) comprises mixing the sample in addition with heparin.

6. The method as claimed in any of the preceding claims, wherein the amino acid substitution Xaa in position 233 and/or position 239 of the GPIbα protein used comprises a valine or a serine residue.

7. The method as claimed in any of the preceding claims, wherein the GPIbα protein used is associated with a solid phase, preferably with a particulate solid phase.

8. The method as claimed in claim 7, wherein the VWF activity is determined on the basis of GPIbα-mediated agglutination of the particulate solid phase.

## Revendications

1. Procédé de détermination de l'activité de clivage du facteur von Willebrand (VWF) de la protéase ADAMTS-13 dans un échantillon, comprenant les stades suivantes :
a) on mélange l'échantillon, dont on pense qu'il contient la protéase ADAMTS-13, à du VWF isolé comme substrat pour obtenir une masse réactionnelle,
b) on fait incuber la masse réactionnelle et
c) on détermine l'activité VWF qui reste dans la masse réactionnelle,
dans lequel
le substrat VWF est constitué d'un VWF multimère, à haut poids moléculaire, qui contient des monomères VWF, qui ont au moins une modification de séquence d' acide aminé, qui fait que le substrat VWF comparé à un substrat VWF constitué de VWF multimère à haut poids moléculaire en monomères VWF de type sauvage, donne une décomposition accélérée par ADAMTS-13,
**caractérisé en ce que**
dans le stade c), la détermination de l'activité VWF restante dans la masse réactionnelle s'effectue par un procédé, dans lequel l'échantillon est mélangé à de la protéine GPlbα isolée, qui comprend une séquence d'acide aminé, qui, comparée à la séquence de type sauvage de la protéine GPlbα humaine (identification de séquence N° 1), contient au moins les restes 1 à 268 d'acide aminé et a, aux positions 233 et 239 respectivement, une substitution Xaa d'acide aminé pour obtenir une masse de test, et dans lequel on ajoute ni de la ristocétine, ni de la botrocétine à la masse de test.

2. Procédé suivant la revendication 1, dans lequel des monomères VWF du substrat VWF composé de VWF multimère, à haut poids moléculaire ont au moins une modification de séquence d'acide aminé du groupe suivant :
P1648S, E1638K, G1505R, S1506L, M1528V, R1569del, R1597W, V1607D, G1609R, G1629E, G1631D, R1597Q, V1499E et Y1584C.

3. Procédé suivant l'une des revendications 1 ou 2, dans lequel le substrat VWF multimère, à haut poids moléculaire est produit par recombinant.

4. Procédé suivant l'une des revendications 1 ou 2, dans lequel le substrat VWF multimère, à haut poids moléculaire est isolé à partir de plasma de donneur de porteurs hétérozygotes ou homozygotes d'une modification appropriée de séquence d'acide aminé VWF.

5. Procédé suivant l'une des revendications précédentes, dans lequel, dans le stade a), on mélange l'échantillon supplémentairement à de l'héparine.

6. Procédé suivant l'une des revendications précédentes, **caractérisé en ce que** la modification Xaa d'acide aminé en position 233 et/ou en position 239 de la protéine GPlbα utilisée est constituée d'un reste de valine ou d'un reste de sérine.

7. Procédé suivant l'une des revendications précédentes, **caractérisé en ce que** la protéine GPlbα utilisée est associée à une phase solide, de préférence à une phase solide en particules.

8. Procédé suivant la revendication 7, **caractérisé en ce que** l'on détermine l'activité VWF au moyen de l'agglutination à médiation GPlbα de la phase solide en particules.


### SEQUENCE LISTING

<110> Siemens Healthcare Diagnostics Products GmbH
<120> Verfahren zur Bestimmung der von Willebrand Faktor-Aktivität in Abwesenheit von Ristocetin und zur Bestimmung der ADAMTS-13-Protease
<130> 2007P59005WE01 - Ma 1289
<140> EP 11002342.1
   <141> 2008-07-03
<150> DE 10 2007 031 708.7
   <151> 2007-07-06
<160> 3
<170> PatentIn version 3.3
<210> 1
   <211> 610
   <212> PRT
   <213> Homo sapiens
<400> 1 
His Pro Ile Cys Glu Val Ser Lys Val Ala Ser His Leu Glu Val Asn
1               5                   10                  15

Cys Asp Lys Arg Asn Leu Thr Ala Leu Pro Pro Asp Leu Pro Lys Asp
            20                  25                  30

Thr Thr Ile Leu His Leu Ser Glu Asn Leu Leu Tyr Thr Phe Ser Leu
        35                  40                  45

Ala Thr Leu Met Pro Tyr Thr Arg Leu Thr Gln Leu Asn Leu Asp Arg
    50                  55                  60

Cys Glu Leu Thr Lys Leu Gln Val Asp Gly Thr Leu Pro Val Leu Gly
65                  70                  75                  80

Thr Leu Asp Leu Ser His Asn Gln Leu Gln Ser Leu Pro Leu Leu Gly
                85                  90                  95

Gln Thr Leu Pro Ala Leu Thr Val Leu Asp Val Ser Phe Asn Arg Leu
            100                 105                 110

Thr Ser Leu Pro Leu Gly Ala Leu Arg Gly Leu Gly Glu Leu Gln Glu
        115                 120                 125

Leu Tyr Leu Lys Gly Asn Glu Leu Lys Thr Leu Pro Pro Gly Leu Leu
    130                 135                 140

Thr Pro Thr Pro Lys Leu Glu Lys Leu Ser Leu Ala Asn Asn Asn Leu
<110> Siemens Healthcare Diagnostics Products GmbH
<120> Verfahren zur Bestimmung der von Willebrand Faktor-Aktivität in Abwesenheit von Ristocetin und zur Bestimmung der ADAMTS-13-Protease
<130> 2007P59005WE01 - Ma 1289
<140> EP 11002342.1
   <141> 2008-07-03
<150> DE 10 2007 031 708.7
   <151> 2007-07-06
<160> 3
<170> PatentIn version 3.3
<210> 1
   <211> 610
   <212> PRT
   <213> Homo sapiens
<400> 1

His Pro Ile Cys Glu Val Ser Lys Val Ala Ser His Leu Glu Val Asn
1               5                   10                  15

Cys Asp Lys Arg Asn Leu Thr Ala Leu Pro Pro Asp Leu Pro Lys Asp
            20                  25                  30

Thr Thr Ile Leu His Leu Ser Glu Asn Leu Leu Tyr Thr Phe Ser Leu
        35                  40                  45

Ala Thr Leu Met Pro Tyr Thr Arg Leu Thr Gln Leu Asn Leu Asp Arg
    50                  55                  60

Cys Glu Leu Thr Lys Leu Gln Val Asp Gly Thr Leu Pro Val Leu Gly
65                  70                  75                  80

Thr Leu Asp Leu Ser His Asn Gln Leu Gln Ser Leu Pro Leu Leu Gly
                85                  90                  95

Gln Thr Leu Pro Ala Leu Thr Val Leu Asp Val Ser Phe Asn Arg Leu
            100                 105                 110

Thr Ser Leu Pro Leu Gly Ala Leu Arg Gly Leu Gly Glu Leu Gln Glu
        115                 120                 125

Leu Tyr Leu Lys Gly Asn Glu Leu Lys Thr Leu Pro Pro Gly Leu Leu
    130                 135                 140

Thr Pro Thr Pro Lys Leu Glu Lys Leu Ser Leu Ala Asn Asn Asn Leu
145                 150                 155                 160